# EUROPÄISCHE PATENTSCHRIFT

(11) **EP 0 567 817 B1**
(45) Veröffentlichungstag und Bekanntmachung des Hinweises auf die Patenterteilung: **10.01.1996**
(21) Anmeldenummer: 93105809.3
(22) Anmeldetag: 08.04.1993
(51) Int. Cl.: A61B 3/04, G02C 13/00

(54) **Messbrille**
Trial frames
Lunettes d'épreuve

(30) Priorität: 29.04.1992 DE 9205768 U
(43) Veröffentlichungstag der Anmeldung: 03.11.1993
(73) Patentinhaber: Oculus Optikgeräte GmbH, D-35582 Wetzlar (DE)
(72) Erfinder: Volkert, Heinz, W-6330 Wetzlar 17 (DE)
(74) Vertreter: Missling, Arne, Dipl.-Ing.

(56) Entgegenhaltungen:
- US-A- 2 256 491
- US-A- 2 432 676
- US-A- 4 673 263

## Beschreibung

Die Erfindung betrifft eine Meßbrille zur Aufnahme von sphärischen, prismatischen und zylindrischen Meßgläsern mit einer Brücke mit zwei hieran befestigten und axial verschiebbaren Hauptkörpern für die Befestigung der Brillenglashalter und einer Nasenhöhenverstellung und mit längen- und höhenverstellbaren Bügeln.

In Meßbrillen der vorstehend genannten Art werden zur Brillenglasbestimmung spezielle Probiergläser eingesetzt, die leicht austauschbar sein müssen. Die Probiergläser müssen darüberhinaus auch drehbar angeordnet sein, um astigmatische Fehler genau in ihrer Achslage auskorrigieren zu können. Des weiteren müssen auch Seiten- und Höhenfehler, die über Prismen korrigiert werden, genau zur Basislage einstellbar sein. Dies setzt voraus, daß die Meßbrillen genau auf den Patienten eingestellt werden können müssen. Das heißt, es muß zum einen der sogenannte Pupillenabstand exakt eingestellt werden, und zwar so, daß die Pupillenmitte eines jeden Auges separat einstellbar ist. Bei den bekannten Meßbrillen ist des weiteren an der Brückenmitte der Meßbrille eine verstellbare Nasenauflage befestigt, die sowohl schwenkbar wie auch in der Höhe einstellbar sein muß. Die Brillenflügel müssen längenveränderbar und in der Höhe verstellbar ausgebildet sein, um exakt den unterschiedlichen Ohrformen angepaßt werden zu können. Diese Meßbrillen müssen des weiteren dafür geeignet sein, bis zu fünf Gläser pro Auge aufnehmen zu können, wobei das Austauschen der Gläser schnell und einfach vonstatten gehen muß.

Bei den bekannten Meßbrillen ist es relativ schwierig und umständlich, die Meßgläser auszutauschen. Des weiteren ist es umständlich, die Nasenhöhenverstellung und den Schwenkmechanismus einzustellen, da hier nicht nur eine Verschiebung, sondern darüberhinaus mit einer Kontermutter eine Arretierung der Nasenhöhenverstellung an der Brücke vorgenommen werden muß. Bei den bekannten Meßbrillen ist darüberhinaus der Bügel mit einem Verschiebemechanismus ausgebildet, der außenliegend offen ist, so daß sich Haare des Patienten hier verklemmen können. Ein weiterer Schwachpunkt der bekannten Brillen besteht darin, daß die Halterung für die Aufnahme der Brillengläser aus einer Dreifachfeder bestand, die bei häufigem Gebrauch relativ früh erlahmt und auf Dauer auch abbrechen konnte.

Der Erfindung liegt die Aufgabe zugrunde, eine Meßbrille der eingangs genannten Art vorzuschlagen, die zumindest bei gleichem Gewicht eine deutlich verbesserte Handhabung gestattet, bei der die Meßgläser leichter ein- und auszuwechseln sind und auch leichter verdrehbar sind, bei der darüberhinaus die Einstellbarkeit der Nasenhöhenverstellung und des Bügels vereinfacht ist und sich keine Haare mehr bei der Verstellung in diesen verklemmen können.

Diese Aufgabe wird mit den Merkmalen des Anspruches 1 gelöst.

Für die Nasenhöhenverstellung wird ein selbsthemmender Antrieb vorgeschlagen, so daß sowohl die Verschwenkung wie auch die Höhenverstellung mit einem einzigen Bedienknopf möglich ist und ein lästiges Anziehen der Kontermutter entfällt. Für die Aufnahme der Meßgläser dienen drei Stege, die entsprechende Aufnahmenuten für die einzelnen Meßgläser aufweisen, wobei einer dieser Stege vorteilhaft federnd ausgebildet ist. Diese Stege sind jeweils in einem Abstand von etwas mehr als 90° auf einem Kreisumfang angeordnet, so daß sichergestellt ist, daß die in etwa fast bis 360° drehbaren Brillengläser nicht aus den Halterungen herausfallen können. Was die Bügel der Meßbrille betrifft, so sind diese in der Höhe verstellbar um ein Gelenk schwenkbar, das senkrecht zur Bügellängsachse verläuft. Dieses Gelenk ist an einem Teleskopteil befestigt, wobei das sich an das Gelenk anschließende Teil das innenliegende Teleskopteil allseitig übergreift. Hierdurch ist sichergestellt, daß im Bereich der Haare keine veränderlichen Spalten entstehen, in die Haare eingeklemmt werden können.

Der Abstand der Brücke zu den Aufnahmen für die Meßgläser wurde bewußt größer als bei den herkömmlichen Brillen gewählt, damit die Gläser einfacher aus ihren Halterungen herausnehmbar sind. Des weiteren wurden auch die Bedienelemente etwas weiter nach außen gelegt, so daß beim Verstellen der Meßbrille der Refraktionist den Patienten in keiner Weise belästigt. Des weiteren wurden die Bedienelemente für die Tastwahrnehmung deutlich verbessert. Die Zylinderelemente und auch die Elemente zur Nasenhöhenverstellung wurden kugelartig ausgeführt. Die Elemente für die Brückenverstellung zur exakten PD-Anpassung wurden zylindrisch ausgebildet.

Die Höhenverstellung der Bügel wurde durch einen komplett neuen Mechanismus ersetzt. Dabei wird ein Bedienrad, auf dessen Innenseite eine Kurvenführung eingespritzt ist, nach rechts bzw. links bewegt, wodurch die Aufwärts- und Abwärtsbewegungen synchron erfolgen.

Die Hauptkörper der Meßbrille können in Kunststoff ausgeführt werden, wobei die Brillenglashalter mit ihren drei Stegen aus einem Aluminium-Druckguß hergestellt werden. Auf diese Stege werden dann Kunststofformteile aufgesteckt, die zur Aufnahme der Meßgläser dienen.

Der Visierstab zur Hornhautscheitelabmessung wurde direkt an dem Hauptkörper an einer vorgesehenen Führung eingeschoben. Die Nasenhöhenverstellung ist mit einem selbsthemmenden Antrieb versehen, so daß über einen Drehknopf die Nasenauflage bequem und individuell angepaßt werden kann. Hierfür dient ein kugeliges Bedienungselement. Läßt man dieses kugelige Bedienungselement los, so ist die Nasenauflage an der eingestellten Stelle direkt arretiert. Ein ergänzendes Kontern, wie beim Stand der Technik, entfällt. Um die Brille parallel vor die Patientenaugen setzen zu können, ist die Nasenhöhenverstellung verschwenkbar, wobei diese Verschwenkung auch bei Loslassen des gleichen Knopfes arretiert wird.

Ein Ausführungsbeispiel der Erfindung ist im folgenden anhand der Zeichnung näher beschrieben. In dieser zeigen
- Fig. 1: eine Vorderansicht einer erfindungsgemäß ausgebildeten Meßbrille,

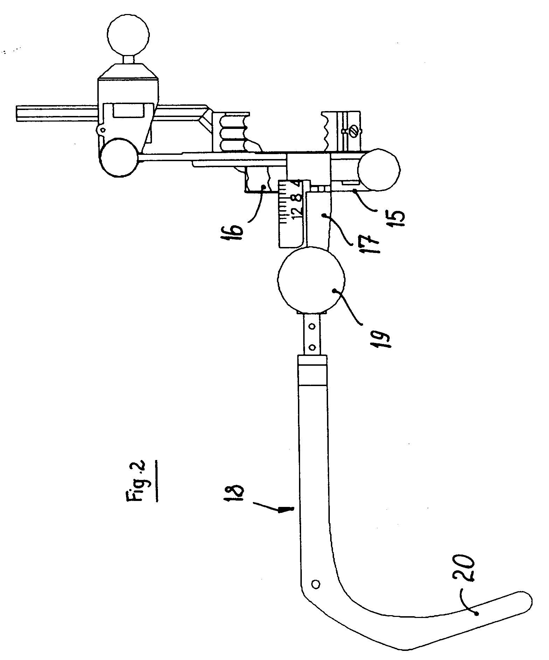

- Fig. 2: eine Rückansicht der Meßbrille und
- Fig. 3: die in Fig. 1 gezeigte Meßbrille mit eingesetzten Meßgläsern.

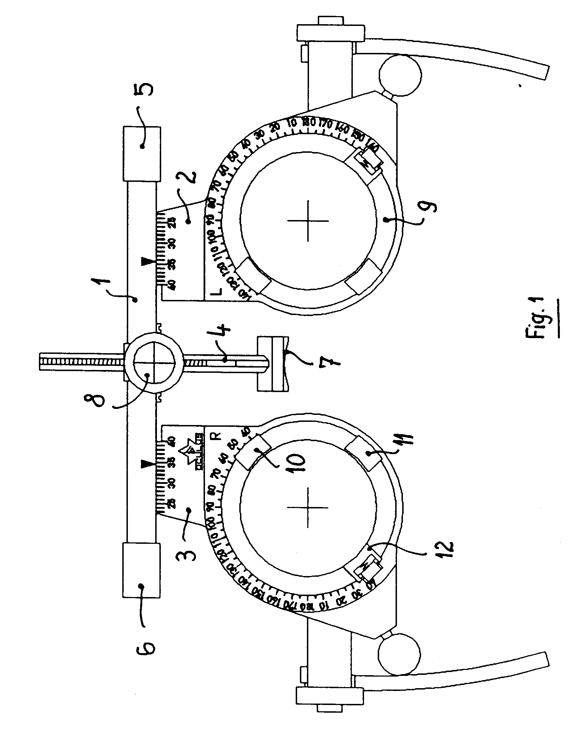

In Fig. 1 bezeichnet 1 die Brücke, an der die beiden Hauptkörper 2, 3 sowie die Nasenhöhenverstellung 4 befestigt sind. Zwei Verstellelemente 5, 6 dienen zur seitlichen Verstellung der Hauptkörper 2, 3 relativ zur Nasenhöhenverstellung. Der Pupillenabstand kann an einer Meßskala abgelesen werden, die am Hauptkörper angeordnet ist und mit diesem relativ zur Brücke 1 verschwenkt wird.

Die Nasenhöhenverstellung 4 ist zum einen schwenkbar an der Meßbrücke 1 befestigt, um die Nasenstütze 7 derart einzustellen, daß die Brille exakt zum Auge ausrichtbar ist. Des weiteren ist die Nasenhöhenverstellung 4 in der Höhe zur Brücke 1 verstellbar, wozu ein Stellknopf 8 dient, der ein selbsthemmendes Getriebe betätigt. Nach Einstellung und Loslassen des Knopfes 8 ist die Nasenhöhenverstellung 4 in ihrer relativen Lage zur Brücke 1 fixiert.

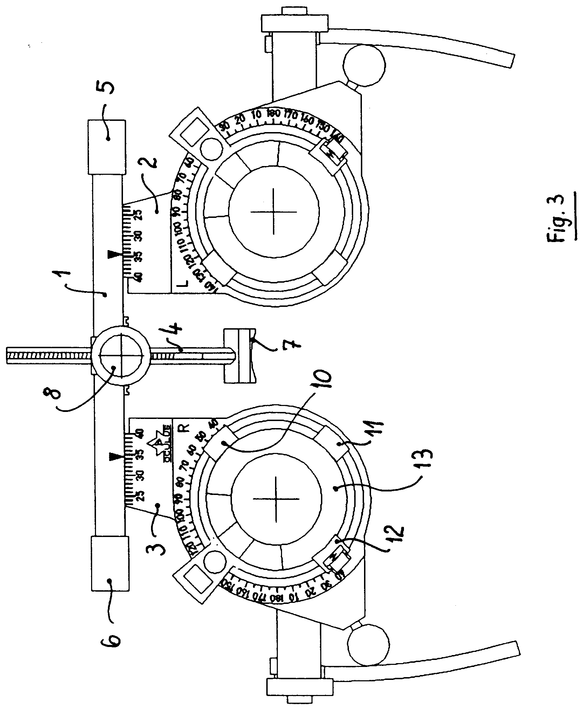

Jeder Hauptkörper 2, 3 nimmt einen drehbaren Ring 9 auf, auf dem drei Stege, 10, 11 und 12 befestigt sind, die zueinander einen Abstand von etwas mehr als 90° aufweisen, so daß das in Fig. 3 gezeigte Meßglas 13 auf etwas mehr als 180° umfaßt wird. Des weiteren ist der Steg 12 federnd ausgebildet, so daß die Meßgläser 13 einfach einsetzbar und sicher gehalten sind.

Zur Verstellung des Ringes 9 dienen Drehantriebe, die über kugelförmige Bedienungselemente 14 betätigbar sind. Auf der Vorderseite der Brille können bis zu drei Meßgläser eingesetzt werden, während auf der Rückseite der Brille zwei Meßgläser einsetzbar sind. Auf der Rückseite der Brille dienen zwei Stege 15, 16 zur Aufnahme der Meßgläser, wobei diese nicht federnd, jedoch mit einem größeren Umfang ausgebildet sind.

An jedem Hauptträger 2, 3 ist ein Stift an dessen Rückseite befestigt, auf dem ein Teil 17 des Bügels 18 aufgeschoben ist. Der Stift greift in einen Bogen des Teiles 17 ein, wobei die Arretierung durch Selbsthemmung erfolgt. Am Ende des Teiles 17 ist ein senkrecht zum Bügel 18 verlaufendes Gelenk 19 angebracht, um das das Bügelende 20 schwenkbar ist.

Die Brücke 1 wurde relativ zu den Meßgläsern 13 hochgesetzt, um zum einen das Einsetzen der Meßgläser 13 zu erleichtern und um die Einstellungen mit einem relativ großen Abstand zum Kopf des Patienten durchführen zu können.

## Patentansprüche

1. Meßbrille zur Aufnahme von sphärischen, prismatischen und zylindrischen Meßgläsern (13) mit einer Brücke (1) mit zwei hieran befestigten und axial verschiebbaren Hauptkörpern (2,3) für die Befestigung der Brillenglashalter und einer Nasenhöhenverstellung (4) und mit Längen- und höhenverstellbaren Bügeln (18), wobei jeder Bügel aus einem vorderen Teil (17) und einem hinteren, in der Länge verstellbaren Teil besteht, welche über ein Gelenk (19) mit horizontaler Achse schwenkbar miteinander verbunden sind, dadurch gekennzeichnet, daß die Brillenglashalterung aus drei auf einem verstellbaren Ring (9) befestigten Stegen (10, 11, 12) besteht, von denen zumindest ein Steg (12) federnd ausgebildet ist, daS die schwenkbar an der Brücke (1) befestigte Nasenhöhenverstellung (4) mit einem selbsthemmenden Antrieb versehen ist, und daß der hintere Teil des Bügels aus teleskopartig inneinander schiebbaren Teilen besteht.

2. Meßbrille nach Anspruch 1, dadurch gekennzeichnet, daß auf den Stegen flexible Kunststoffteile aufgesetzt sind, die Aufnahmen für die Meßgläser (13) aufweisen.

3. Meßbrille nach Anspruch 1 oder 2, dadurch gekennzeichnet, daß die Bügelneigeeinstellung über ein Bedienelement erfolgt, das eine Kurve aufweist, die bei einer Drehung nach rechts den Bügel nach unten und bei einer Drehung nach links den Bügel nach oben bewegt.

4. Meßbrille nach einem der Ansprüche 1 bis 3, dadurch gekennzeichnet, daß die Bedienelemente zur Verstellung von Zylinderachse bzw. Prismenbasislage und zur Nasenhöhenverstellung kugelförmig ausgebildet sind.

5. Meßbrille nach einem der Ansprüche 1 bis 4, dadurch gekennzeichnet, daß die Bedienelemente an der Brücke zur Verstellung des Pupillenabstandes zylindrisch ausgeführt sind.

6. Meßbrille nach einem der Ansprüche 1 bis 5, dadurch gekennzeichnet, daß der drehbare Ring (9) aus Metall ausgebildet und in einer Kunststofführung gehaltert ist.

## Claims

1. Test spectacles for receiving spherical, prismatic and cylindrical test lenses (13), having a bridge (1) with two main bodies (2, 3) which are secured thereto, are axially displaceable and are for securing the spectacle lens holders and a nose vertical adjustment device (4), and having longitudinally and vertically adjustable temples (18), each temple being composed of a front part (17) and a rear longitudinally-adjustable part which are pivotably connected to one another via a joint (19) with a horizontal axis, characterised in that the spectacle lens holding device is composed of three flanges (10, 11, 12) secured on an adjustable ring (9), at least one flange (12) being resilient, in that the nose vertical adjustment device (4) pivotably secured to the bridge (1) is provided with a self-locking drive, and in that the rear part of the temple is composed of parts which can be slid into one another in a telescopic manner.

2. Test spectacles in accordance with Claim 1, characterised in that on the flanges there are placed flexible plastics parts having receivers for the test lenses (13).

3. Test spectacles in accordance with Claim 1 or 2, characterised in that adjustment of the temple inclination takes place via an operating element having a cam which moves the temple downwards upon rotation to the right and moves the temple upwards upon rotation to the left.

4. Test spectacles in accordance with any one of Claims 1 to 3, characterised in that the operating elements for adjustment of cylinder axis or prism base position and for the nose vertical adjustment device are spherical.

5. Test spectacles in accordance with any one of Claims 1 to 4, characterised in that the operating elements at the bridge for adjustment of the pupil spacing are cylindrical.

6. Test spectacles in accordance with any one of Claims 1 to 5, characterised in that the rotatable ring (9) is of metal and is secured in a plastics guiding device.

## Revendications

1. Lunettes d'essai, destinées à supporter des verres de mesure (13) sphériques, prismatiques et cylindriques, comportant
un pont (1) qui porte deux corps principaux (2, 3) fixés sur ce pont et déplacés axialement, pour la fixation des supports de verres de lunettes et d'un élément de réglage (4) pour la hauteur du nez,
et des branches (18) réglables en longueur et en hauteur, chaque branche étant constituée d'une pièce avant (17) et d'une pièce arrière réglable en longueur, lesdites pièces étant reliées l'une à l'autre, de manière à pouvoir pivoter, par l'intermédiaire d'une articulation (19) d'axe horizontal,
caractérisées en ce que la structure des supports de verres de lunettes est constituée de trois curseurs (10, 11, 12) fixés sur un anneau (9) réglable, au moins un curseur (12) étant élastique,
en ce que l'élément de réglage (4) pour la hauteur du nez, fixé sur le pont (1) avec possibilité de pivoter est pourvu d'un entraînement autobloquant,
et en ce que la pièce arrière de la branche est constituée d'éléments glissant les uns dans les autres de manière télescopique.

2. Lunettes d'essai selon la revendication 1, caractérisées en ce que des pièces en matière plastique souple, présentant des logements pour les verres d'essai (13), sont posées sur les curseurs.

3. Lunettes d'essai selon la revendication 1 ou 2, caractérisées en ce que le réglage de l'inclinaison des branches, s'opère grâce à un organe de réglage, présentant une courbe dont une rotation à droite déplace la branche vers le bas et une rotation à gauche déplace la branche vers le haut.

4. Lunettes d'essai selon l'une des revendications 1 à 3, caractérisées en ce que les organes de réglage destinés au réglage des axes de cylindre ou de la position prismatique de base et au réglage pour la hauteur du nez sont formés de billes.

5. Lunettes d'essai selon l'une des revendications 1 à 4, caractérisées en ce que les organes de réglage situés sur le pont pour le réglage de l'écartement entre pupilles sont de forme cylindrique.

6. Lunettes d'essai selon l'une des revendications 1 à 5, caractérisées en ce que l'anneau (9) rotatif est constitué de métal et est maintenu dans un guide en matière plastique.
